(19) Europäisches Patentamt
European Patent Office
Office européen des brevets

(11) EP 3 897 539 B1

(12) EUROPEAN PATENT SPECIFICATION

(45) Date of publication and mention of the grant of the patent:
**04.01.2023 Bulletin 2023/01**

(21) Application number: **19827700.6**

(22) Date of filing: **18.12.2019**

(51) International Patent Classification (IPC):
***A61K 8/33*** *(2006.01)* ***A61K 8/73*** *(2006.01)*
***A61Q 5/06*** *(2006.01)* ***A61Q 5/10*** *(2006.01)*

(52) Cooperative Patent Classification (CPC):
**A61K 8/73; A61K 8/33; A61K 8/737; A61Q 5/065; A61Q 5/10**

(86) International application number:
**PCT/EP2019/085871**

(87) International publication number:
**WO 2020/127438 (25.06.2020 Gazette 2020/26)**

(54) **DYE COMPOSITION COMPRISING AT LEAST ONE POLYSACCHARIDE-TYPE POLYMER AND PROCESS FOR DYEING KERATIN FIBERS USING SAME**

FARBSTOFFZUSAMMENSETZUNG MIT MINDESTENS EINEM POLYSACCHARID-POLYMER UND VERFAHREN ZUM FÄRBEN VON KERATINFASERN UNTER VERWENDUNG DIESER ZUSAMMENSETZUNG

COMPOSITION COLORANTE COMPRENANT AU MOINS UN POLYMÈRE DE TYPE POLYSACCHARIDE ET PROCEDE DE COLORATION DES FIBRES KERATINIQUES LA METTANT EN OEUVRE.

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.12.2018 FR 1873800**

(43) Date of publication of application:
**27.10.2021 Bulletin 2021/43**

(73) Proprietor: **L'OREAL**
**75008 Paris (FR)**

(72) Inventors:
- **LAFUMA, Aurélie**
  **93400 SAINT-OUEN (FR)**
- **LALLEMAN, Boris**
  **93400 SAINT-OUEN (FR)**

(74) Representative: **L'Oreal**
**Service D.I.P.I.**
**9, rue Pierre Dreyfus**
**92110 Clichy (FR)**

(56) References cited:
**EP-A1- 1 757 326**
**EP-A1- 1 935 455**
**EP-A2- 1 669 104**
**WO-A1-2011/121008**
**FR-A1- 3 060 319**

- **DATABASE GNPD [Online] MINTEL; 24 July 2017 (2017-07-24), anonymous: "Coloring Shampoo", XP055623415, retrieved from www.gnpd.com Database accession no. 4977629**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 897 539 B1

## Description

The present invention relates to a composition for dyeing keratin fibers, preferably human keratin fibers such as the hair.

More specifically, one subject of the present invention is a dye composition comprising at least one polysaccharide-type polymer, at least one fatty ether that is solid at room temperature and atmospheric pressure and at least one dye and/or dye precursor.

It likewise relates to a process for dyeing keratin fibers using such a composition and also to the use of the composition according to the invention.

Many people have sought for a long time to modify the color of their hair and in particular to mask their white hair.

It is notably known practice to dye keratin fibers, in particular human keratin fibers, with dye compositions containing oxidation dye precursors, which are generally known as oxidation bases. These oxidation bases are colorless or weakly colored compounds which, when combined with oxidizing products, may give rise to colored compounds via a process of oxidative condensation.

The shades obtained with these oxidation bases may be modified by combining them with couplers or color modifiers. The variety of molecules used as oxidation bases and couplers allows a wide range of colors to be obtained.

Another well-known method consists in obtaining "semipermanent" dyeing by applying to the keratin fibers direct dyes which are colored and coloring molecules that have an affinity for said fibers.

The direct dyes conventionally used are chosen from nitrobenzene, anthraquinone, nitropyridine, azo, xanthene, acridine, azine and triarylmethane direct dyes. The chemical species may be nonionic, anionic (acidic dyes) or cationic (basic dyes). The direct dyes may also be natural dyes.

Conventional direct dyeing processes consist in applying to the keratin fibers dye compositions comprising direct dyes. After application, a leave-on time is observed so as to allow the dye molecules to penetrate by diffusion into the fibers. On conclusion of the process, the fibers are rinsed.

In contrast with oxidation dyeing, these direct dyeing processes have a tendency to better protect the integrity of the fibers. The resulting colorings are generally chromatic, but are, however, only semi-temporary. The nature of the interactions that bind the direct dyes to the keratin fibers and their desorption from the surface and/or the core of the fiber are responsible for their weak dyeing power. It is thus possible to observe less favorable kinetics, a reduced intensity of the shade obtained, a worse homogeneity of the color from one fiber to the next and/or depending on the location on the fiber (root/tip), etc.

Thus, there is a real need to provide dye compositions that do not have the abovementioned drawbacks, i.e. which make it possible in particular to result in colorings that have good properties, notably in terms of chromaticity, power, intensity and selectivity, which are persistent with respect to shampoo washing and/or which do not impair the cosmetic properties of the keratin fibers.

Thus, one subject of the present invention is a composition comprising:

- at least one polysaccharide-type polymer,
- at least one fatty ether that is solid at room temperature and atmospheric pressure, of formula R-O-R' wherein R and R', which are identical or different, denote a linear or branched alkyl or alkenyl radical, R and R' comprising at least 12 carbon atoms, and
- at least one direct dye and/or one dye precursor.

Another subject of the invention is a process for dyeing keratin fibers, in particular human keratin fibers such as the hair, using such a composition.

Lastly, the invention relates to the use of the composition according to the invention for dyeing keratin fibers, in particular human keratin fibers such as the hair.

The composition according to the invention makes it possible to obtain powerful, chromatic and sparingly selective colorings, while giving the treated fibers good cosmetic properties, notably in terms of sheen and while limiting the degradation thereof.

The compositions in accordance with the present invention furthermore have a texture that is particularly well suited for use in dyeing the hair. Specifically they are unctuous, sufficiently thick and opacified or nacreous for an accurate, rapid and easy application, with good removal on rinsing, without leaving the hair greasy, without however running beyond the areas of the head of hair that it is desired to treat.

Other features and advantages of the invention will emerge more clearly on reading the description and the examples that follow.

For the purposes of the present invention and unless otherwise indicated:

- for the purposes of the present invention, the term *"direct dye"* means colored species - natural and/or synthetic dyes - which are soluble in the cosmetic medium; these are dyes which will diffuse superficially on the keratin fibers;

- for the purposes of the present invention, the term *"dye precursor*" means means any colorless molecule which, under the action of an oxidizing agent, gives a colored species which colors the keratin fibers; preferably these are oxidation dyes; and

- the term "*alkyl*" means a linear or branched saturated hydrocarbon chain which comprises between 1 and 6 carbon atoms, and

- the term "*alkenyl*" means a hydrocarbon chain comprising one or more conjugated or non-conjugated double bonds, which comprises between 2 and 6 carbon atoms, and

- the expression *"at least one"* is equivalent to *"one or more";* and

- the limits of a range of values are included in that range, in particular in the expressions *"between"* and *"ranging from ... to ..."*.

***i) polysaccharide-type polymer***

The composition according to the invention contains one or more polysaccharide-type polymers.

The polysaccharide-type polymers useful for the invention are polymers which may be of natural or synthetic origin.

Preferably, the polysaccharide-type polymers are thickening polymers. The term *"thickening polymer*" means a polymer which, when introduced at 1% by weight in an aqueous solution or an aqueous-alcoholic solution containing 30% ethanol, and at pH = 7, or in an oil chosen from liquid petroleum jelly, isopropyl myristate or cyclopentadimethylsiloxane, makes it possible to achieve a viscosity of at least 100 cps and preferably of at least 500 cps, at 25°C and at a shear rate of 1 $s^{-1}$. This viscosity may be measured using a cone/plate viscometer (Haake R600 rheometer or the like).

The polysaccharide polymers useful for the invention are cationic, nonionic, anionic or amphoteric polymers, preferably cationic, nonionic or anionic polymers, better still nonionic polymers.

The term *"polysaccharide polymers"* means polymers bearing sugar units.

For the purposes of the present invention, the term "sugar unit" means a unit derived from a carbohydrate of formula $C_n(H_2O)_{n-1}$ or $(CH_2O)_n$, which may be optionally modified by substitution and/or by oxidation and/or by dehydration.

As sugar units of the polymers useful for the invention are preferably derived from the following sugars: glucose, galactose, arabinose, rhamnose, mannose, xylose, fucose, anhydrogalactose, galacturonic acid, glucuronic acid, mannuronic acid, galactose sulfate, anhydrogalactose sulfate and fructose.

Mention may in particular be made, as polysaccharide polymers, of those derived from native gums such as:

a) tree or shrub exudates, including:

- gum arabic (branched polymer of galactose, arabinose, rhamnose and glucuronic acid);
- ghatti gum (polymer derived from arabinose, galactose, mannose, xylose and glucuronic acid);
- karaya gum (polymer derived from galacturonic acid, galactose, rhamnose and glucuronic acid);
- gum tragacanth (polymer of galacturonic acid, galactose, fucose, xylose and arabinose);

b) gums derived from algae, including:

- agar (polymer derived from galactose and anhydrogalactose);
- alginates (polymers of mannuronic acid and of glucuronic acid);
- carrageenans and furcellerans (polymers of galactose sulfate and of anhydrogalactose sulfate);

c) gums derived from seeds or tubers, including:

- guar gum (polymer of mannose and galactose);
- locust bean gum (polymer of mannose and galactose);
- fenugreek gum (polymer of mannose and galactose);
- tamarind gum (polymer of galactose, xylose and glucose);
- konjac gum (polymer of glucose and mannose);

d) microbial gums, including:

- xanthan gum (polymer of glucose, mannose acetate, mannose/pyruvic acid and glucuronic acid);
- gellan gum (polymer of partially acylated glucose, rhamnose and glucuronic acid);
- scleroglucan gum (glucose polymer);

e) plant extracts, including:

- cellulose (glucose polymer);
- starch (glucose polymer);
- and inulin;
- pectin.

These polymers may be physically or chemically modified. As physical treatment, mention may notably be made of the temperature.

Chemical treatments that may be mentioned include esterification, etherification, amidation and oxidation reactions. These treatments make it possible to lead to polymers that may in particular be nonionic, anionic or amphoteric.

Preferably, these chemical or physical treatments are applied to guar gums, locust bean gums, starches and celluloses.

The nonionic guar gums that may be used according to the invention may be modified with $C_1$-$C_6$ (poly)hydroxyalkyl groups.

Among the $C_1$-$C_6$ (poly)hydroxyalkyl groups, mention may be made, by way of example, of hydroxymethyl, hydroxyethyl, hydroxypropyl and hydroxybutyl groups.

These guar gums are well known from the prior art and may be prepared, for example, by reacting corresponding alkene oxides, for instance propylene oxides, with the guar gum so as to obtain a guar gum modified with hydroxypropyl groups.

The degree of hydroxyalkylation preferably varies from 0.4 to 1.2 and corresponds to the number of alkylene oxide molecules consumed by the number of free hydroxyl functions present on the guar gum.

Such nonionic guar gums optionally modified with hydroxyalkyl groups are sold, for example, under the trade names Jaguar HP8, Jaguar HP60, Jaguar HP105 and Jaguar HP120 by the company Rhodia Chimie.

The polysaccharide polymers may be cellulose-based polymers.

Thus, the cellulose-based polymers may be chosen from unsubstituted celluloses, including those in a microcrystalline form, and cellulose ethers.

Among these cellulose-based polymers, cellulose ethers, cellulose esters and cellulose ester ethers are distinguished.

Among the cellulose esters are inorganic esters of cellulose (cellulose nitrates, sulfates, phosphates, etc.), organic esters of cellulose (cellulose monoacetates, triacetates, amidopropionates, acetatebutyrates, acetatepropionates and acetatetrimellitates, etc.), and mixed organic/inorganic esters of cellulose, such as cellulose acetatebutyrate sulfates and cellulose acetatepropionate sulfates. Among the cellulose ester ethers, mention may be made of hydroxypropylmethylcellulose phthalates and ethylcellulose sulfates.

Among the nonionic cellulose ethers without a $C_{10}$-$C_{30}$ fatty chain, i.e. which are *"non-associative",* mention may be made of ($C_1$-$C_4$)alkylcelluloses, such as methylcelluloses and ethylcelluloses (for example, Ethocel standard 100 Premium from Dow Chemical); (poly)hydroxy($C_1$-$C_4$)alkylcelluloses, such as hydroxymethylcelluloses, hydroxyethylcelluloses (for example, Natrosol 250 HHR provided by Aqualon) and hydroxypropylcelluloses (for example, Klucel EF from Aqualon); mixed (poly)hydroxy($C_1$-$C_4$)alkyl-($C_1$-$C_4$)alkylcelluloses celluloses, such as hydroxypropylmethylcelluloses (for example, Methocel E4M from Dow Chemical), hydroxyethylmethylcelluloses, hydroxyethylethylcelluloses (for example, Bermocoll E 481 FQ from Akzo Nobel) and hydroxybutylmethylcelluloses.

Among the anionic cellulose ethers, mention may be made of (poly)carboxy($C_1$-$C_4$)alkylcelluloses and salts thereof. By way of example, mention may be made of carboxymethylcelluloses, carboxymethylmethylcelluloses (for example Blanose 7M from the company Aqualon) and carboxymethylhydroxyethylcelluloses, and the sodium salts thereof.

Among the cationic cellulose ethers, mention may be made of cationic cellulose derivatives such as cellulose copolymers or cellulose derivatives grafted with a water-soluble quaternary ammonium monomer, and described in particular in patent US 4 131 576, such as (poly)hydroxy($C_1$-$C_4$)alkyl celluloses, for instance hydroxymethyl-, hydroxyethyl- or hydroxypropylcelluloses grafted especially with a methacryloylethyltrimethylammonium, methacrylamidopropyltrimethylammonium or dimethyldiallylammonium salt. The commercial products corresponding to this definition are more particularly the products sold under the names Celquat® L 200 and Celquat® H 100 by the company National Starch.

Preferably, the polysaccharide(s) according to the invention are chosen from microbial gums.

The microbial gums can be chosen from scleroglucan gums, gellan gums, pullulan gums, curdlan gums, xanthan gums, grifolan gums, lentinan gums, schizophyllan gums, spirulinan gums and krestin gums.

Mention may in particular be made of the scleroglucan gums produced by Sclerotium rolfsii, the gellan gums produced by Pseudomonas elodea or Sphingomonias, the pullulan gums produced by Aureobacidium pullulens, the curdlan gums produced by Alcaligenes of Faecalis myxogenes type, the xanthan gums produced by numerous organisms, including Leuconostoc mesenteroides and Leuconostoc dextrantum, the grifolan gums produced by Grifola frondara, the lentinan gums produced by Lentinus edodes, the schizophyllan gums produced by Schizophyllum commine, the spirulinan gums produced by Spirulina sybsyla and the krestin gums produced by Coriates versicolor.

More preferentially, the polysaccharide(s) according to the invention is (are) chosen from scleroglucan gums.

The scleroglucans used in accordance with the invention are neutral polysaccharides preferably corresponding to formula (I):

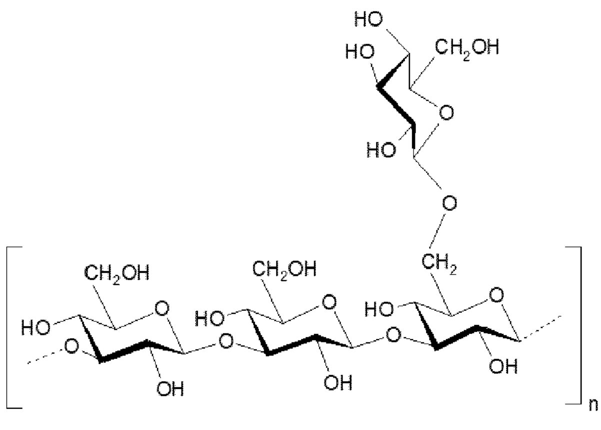

(I)

where the degree of polymerization n ranges from 500 to 1600.

According to the invention, use is advantageously made of scleroglucans of microbial origin, obtained for example by aerobic fermentation of a glucose-containing medium by a fungus of the Sclerotium type having the structure of a D-glucopyranose homopolymer.

Examples of scleroglucan gums that may be used in the present invention are, in a non-limiting manner, the products sold under the name Actigum CS, in particular Actigum CS 11 by the company Sanofi Bio Industries and under the name Amigum or Amigel by the company Alban Müller International.

Other scleroglucans, such as the one treated with glyoxal described in French patent application No. 2,633,940, may also be used.

Use will in particular be made of scleroglucan gums having the INCI name Sclerotium gum.

Preferably, the polysaccharide(s) according to the invention are chosen from microbial gums, hydroxyalkyl guars and (hydroxy)($C_1$-$C_6$)alkyl celluloses.

The polysaccharide polymer(s) is or are generally present in the composition according to the invention in a total content ranging from 0.001% to 10% by weight and preferably from 0.05% to 5%, and more preferentially from 0.1% to 3% by weight, better still from 0.5% to 2.5% by weight, relative to the total weight of the composition.

### *ii) Solid fatty ether*

The composition according to the invention comprises one or more fatty ethers of formula R-O-R' wherein R and R', which are identical or different, denote a linear or branched alkyl or alkenyl radical, R and R' comprising at least 12 carbon atoms. The ether of use in the present invention is a solid fatty ether.

The term *"solid fatty ether"* means a fatty ether that is solid at room temperature and at atmospheric pressure (25°C, 1 atm).

Preferably, the ether is chosen from compounds for which the R and R' radicals, which are identical or different, denote a $C_{12}$-$C_{40}$, preferably $C_{12}$-$C_{30}$, more preferentially $C_{14}$-$C_{24}$ linear or branched alkyl or alkenyl radical.

In accordance with one particular embodiment of the invention, the R and R' radicals, which are identical or different, are radicals that are derived from oleyl (C18), lauryl (C12), palmityl (C16), myristyl (C14), behenyl (C22), stearyl (C18), linoleyl (C18), linolenyl (C18), arachidyl (C20) alcohols.

Preferably, R and R' represent a $C_{12}$-$C_{40}$, preferably $C_{12}$-$C_{30}$, more preferentially $C_{14}$-$C_{24}$ linear alkyl radical.

More particularly, R and R' are identical.

Preferentially, R and R' are identical and represent a $C_{14}$-$C_{24}$ linear alkyl radical, better still R and R' denote a $C_{16}$-$C_{22}$ linear alkyl radical, even better still a stearyl radical.

Preferentially, the ethers that can be used according to the invention are saturated.

Preferentially, the ethers that can be used according to the invention have a melting point above 50°C.

The ethers that can be used according to the invention may be soluble or insoluble in the compositions, but preferably they are insoluble.

These compounds may be prepared according to the process described in patent application DE 41 27 230.

According to one particular embodiment, the ether is distearyl ether or dioctadecyl ether, and corresponds to the formula above in which R and R' represent a $CH_3(CH_2)_{17}$- group.

The composition according to the invention has more particularly a total content of solid fatty ether(s) of between 0.1% and 40% by weight relative to the weight of the dye composition, preferably between 1% and 25% by weight, preferentially between 5% and 15% by weight relative to the weight of the dye composition.

### *iii) Direct dye and/or dye precursor*

The composition according to the invention comprises one or more direct dyes and/or dye precursors.

*a) Dye precursors*

The dye precursors are generally chosen from oxidation bases, optionally combined with coupling agents.

By way of example, the oxidation bases are chosen from para-phenylenediamines, bis(phenyl)alkylenediamines, para-aminophenols, ortho-aminophenols and heterocyclic bases, and the corresponding addition salts.

Among the para-phenylenediamines that may be mentioned are, for example, para-phenylenediamine, para-toluenediamine, 2-chloro-para-phenylenediamine, 2,3-dimethyl-para-phenylenediamine, 2,6-dimethyl-para-phenylenediamine, 2,6-diethyl-para-phenylenediamine, 2,5-dimethyl-para-phenylenediamine, N,N-dimethyl-para-phenylenediamine, N,N-diethyl-para-phenylenediamine, N,N-dipropyl-para-phenylenediamine, 4-amino-N,N-diethyl-3-methylaniline, N,N-bis(β-hydroxyethyl)-para-phenylenediamine, 4-N,N-bis(β-hydroxyethyl)amino-2-methylaniline, 4-N,N-bis(β-hydroxyethyl)amino-2-chloroaniline, 2-β-hydroxyethyl-para-phenylenediamine, 2-methoxymethyl-para-phenylenediamine, 2-fluoro-para-phenylenediamine, 2-isopropyl-para-phenylenediamine, N-(β-hydroxypropyl)-para-phenylenediamine, 2-hydroxymethyl-para-phenylenediamine, N,N-dimethyl-3-methyl-para-phenylenediamine, N-ethyl-N-(β-hydroxyethyl)-para-phenylenediamine, N-(β,γ-dihydroxypropyl)-para-phenylenediamine, N-(4'-aminophenyl)-para-phenylenediamine, N-phenyl-para-phenylenediamine, 2-β-hydroxyethyloxy-para-phenylenediamine, 2-β-acetylaminoethyloxy-para-phenylenediamine, N-(β-methoxyethyl)-para-phenylenediamine, 4-aminophenylpyrrolidine, 2-thienyl-para-phenylenediamine, 2-β-hydroxyethylamino-5-aminotoluene and 3-hydroxy-1-(4'-aminophenyl)pyrrolidine, and the corresponding addition salts with an acid.

Among the para-phenylenediamines mentioned above, para-phenylenediamine, para-toluenediamine, 2-isopropyl-para-phenylenediamine, 2-β-hydroxyethyl-para-phenylenediamine, 2-β-hydroxyethyloxy-para-phenylenediamine, 2,6-dimethyl-para-phenylenediamine, 2,6-diethyl-para-phenylenediamine, 2,3-dimethyl-para-phenylenediamine, N,N-bis(β-hydroxyethyl)-para-phenylenediamine, 2-chloro-para-phenylenediamine and 2-β-acetylaminoethyloxy-para-phenylenediamine, and the corresponding addition salts with an acid, are particularly preferred.

Among the bis(phenyl)alkylenediamines that may be mentioned, for example, are N,N'-bis(β-hydroxyethyl)-N,N'-bis(4'-aminophenyl)-1,3-diaminopropanol, N,N'-bis(β-hydroxyethyl)-N,N'-bis(4'-aminophenyl)ethylenediamine, N,N'-bis(4-aminophenyl)tetramethylenediamine, N,N'-bis(β-hydroxyethyl)-N,N'-bis(4-aminophenyl)tetramethylenediamine, N,N'-bis(4-methylaminophenyl)tetramethylenediamine, N,N'-bis(ethyl)-N,N'-bis(4'-amino-3'-methylphenyl)ethylenediamine and 1,8-bis(2,5-diaminophenoxy)-3,6-dioxaoctane, and the corresponding addition salts.

Among the para-aminophenols that are mentioned are, for example, para-aminophenol, 4-amino-3-methylphenol, 4-amino-3-fluorophenol, 4-amino-3-chlorophenol, 4-amino-3-hydroxymethylphenol, 4-amino-2-methylphenol, 4-amino-2-hydroxymethylphenol, 4-amino-2-methoxymethylphenol, 4-amino-2-aminomethylphenol, 4-amino-2-(β-hydroxyethylaminomethyl)phenol and 4-amino-2-fluorophenol, and the corresponding addition salts with an acid.

Among the ortho-aminophenols that may be mentioned, for example, are 2-aminophenol, 2-amino-5-methylphenol, 2-amino-6-methylphenol and 5-acetamido-2-aminophenol, and the corresponding addition salts.

Among the heterocyclic bases that may be mentioned, for example, are pyridine, pyrimidine and pyrazole derivatives.

Among the pyridine derivatives that may be mentioned are the compounds described, for example, in patents GB 1 026 978 and GB 1 153 196, for example 2,5-diaminopyridine, 2-(4-methoxyphenyl)amino-3-aminopyridine and 3,4-diaminopyridine, and the corresponding addition salts.

Other pyridine oxidation bases that are useful in the present invention are the 3-aminopyrazolo[1,5-a]pyridine oxidation bases or the corresponding addition salts described, for example, in patent application FR 2 801 308. Examples that may be mentioned include pyrazolo[1,5-a]pyrid-3-ylamine, 2-acetylaminopyrazolo[1,5-a]pyrid-3-ylamine, 2-(morpholin-4-yl)pyrazolo[1,5-a]pyrid-3-ylamine, 3-aminopyrazolo[1,5-a]pyridine-2-carboxylic acid, 2-methoxypyrazolo[1,5-a]pyrid-3-ylamine, (3-aminopyrazolo[1,5-a]pyrid-7-yl)methanol, 2-(3-aminopyrazolo[1,5-a]pyrid-5-yl)ethanol, 2-(3-aminopyrazolo[1,5-a]pyrid-7-yl)ethanol, (3-aminopyrazolo[1,5-a]pyrid-2-yl)methanol, 3,6-diaminopyrazolo[1,5-a]pyridine, 3,4-diaminopyrazolo[1,5-a]pyridine, pyrazolo[1,5-a]pyridine-3,7-diamine, 7-(morpholin-4-yl)pyrazolo[1,5-a]pyrid-3-ylamine, pyrazolo[1,5-a]pyridine-3,5-diamine, 5-(morpholin-4-yl)pyrazolo[1,5-a]pyrid-3-ylamine, 2-[(3-aminopyrazolo[1,5-a]pyrid-5-yl)(2-hydroxyethyl)amino]ethanol, 2-[(3-aminopyrazolo[1,5-a]pyrid-7-yl)(2-hydroxyethyl)amino]ethanol, 3-aminopyrazolo[1,5-a]pyridin-5-ol, 3-aminopyrazolo[1,5-a]pyridin-4-ol, 3-aminopyrazolo[1,5-a]pyridin-6-ol, 3-aminopyrazolo[1,5-a]pyridin-7-ol, 2-β-hydroxyethoxy-3-aminopyrazolo[1,5-a]pyridine; 2-(4-dimethylpiperazinium-1-yl)-3-aminopyrazolo[1,5-a]pyridine; and 2-[(3-aminopyrazolo[1,5-a]pyridin-2-yl)oxy]ethanol, and the corresponding addition salts.

Among the pyrimidine derivatives that may be mentioned are the compounds described, for example, in patents DE 2359399; JP 88-169571; JP 05-63124; EP 0770375 or patent application WO 96/15765, such as 2,4,5,6-tetraaminopyrimidine, 4-hydroxy-2,5,6-triaminopyrimidine, 2-hydroxy-4,5,6-triaminopyrimidine, 2,4-dihydroxy-5,6-diaminopyrimidine, 2,5,6-triaminopyrimidine and the addition salts thereof and the tautomeric forms thereof, when a tautomeric equilibrium exists.

Among the pyrazole derivatives that may be mentioned are the compounds described in patents DE 3843892 and DE 4133957 and patent applications WO 94/08969, WO 94/08970, FR-A-2 733 749 and DE 195 43 988, for instance 4,5-diamino-1-methylpyrazole, 4,5-diamino-1-(β-hydroxyethyl)pyrazole, 3,4-diaminopyrazole, 4,5-diamino-1-(4'-chlorobenzyl)pyrazole, 4,5-diamino-1,3-dimethylpyrazole, 4,5-diamino-3-methyl-1-phenylpyrazole, 4,5-diamino-1-methyl-3-phenylpyrazole, 4-amino-1,3-dimethyl-5-hydrazinopyrazole, 1-benzyl-4,5-diamino-3-methylpyrazole, 4,5-diamino-3-*tert*-butyl-1-methylpyrazole, 4,5-diamino-1-*tert*-butyl-3-methylpyrazole, 4,5-diamino-1-(β-hydroxyethyl)-3-methylpyrazole, 4,5-diamino-1-ethyl-3-methylpyrazole, 4,5-diamino-1-ethyl-3-(4'-methoxyphenyl)pyrazole, 4,5-diamino-1-ethyl-3-hydroxymethylpyrazole, 4,5-diamino-3-hydroxymethyl-1-methylpyrazole, 4,5-diamino-3-hydroxymethyl-1-isopropylpyrazole, 4,5-diamino-3-methyl-1-isopropylpyrazole, 4-amino-5-(2'-aminoethyl)amino-1,3-dimethylpyrazole, 3,4,5-triaminopyrazole, 1-methyl-3,4,5-triaminopyrazole, 3,5-diamino-1-methyl-4-methylaminopyrazole and 3,5-diamino-4-(β-hydroxyethyl)amino-1-methylpyrazole, and the corresponding addition salts. Use may also be made of 4,5-diamino-1-(β-methoxyethyl)pyrazole.

A 4,5-diaminopyrazole will preferably be used and even more preferentially 4,5-diamino-1-(β-hydroxyethyl)pyrazole and/or a corresponding salt.

The pyrazole derivatives that may also be mentioned include diamino-N,N-dihydropyrazolopyrazolones and in particular those described in patent application FR-A-2 886 136, such as the following compounds and the corresponding addition salts: 2,3-diamino-6,7-dihydro-1H,5H-pyrazolo[1,2-a]pyrazol-1-one, 2-amino-3-ethylamino-6,7-dihydro-1H,5H-pyrazolo[1 ,2-a]pyrazol-1-one, 2-amino-3-isopropylamino-6,7-dihydro-1H,5H-pyrazolo[1 ,2-a]pyrazol-1-one, 2-amino-3-(pyrrolidin-1-yl)-6,7-dihydro-1H,5H-pyrazolo[1 ,2-a]pyrazol-1-one, 4,5-diamino-1,2-dimethyl-1,2-dihydropyrazol-3-one, 4,5-diamino-1,2-diethyl-1,2-dihydropyrazol-3-one, 4,5-diamino-1,2-bis(2-hydroxyethyl)-1,2-dihydropyrazol-3-one, 2-amino-3-(2-hydroxyethyl)amino-6,7-dihydro-1H,5H-pyrazolo[1,2-a]pyrazol-1-one, 2-amino-3-dimethylamino-6,7-dihydro-1H,5H-pyrazolo[1,2-a]pyrazol-1-one, 2,3-diamino-5,6,7,8-tetrahydro-1H,6H-pyridazino[1,2-a]pyrazol-1-one, 4-amino-1,2-diethyl-5-(pyrrolidin-1-yl)-1,2-dihydropyrazol-3-one, 4-amino-5-(3-dimethylaminopyrrolidin-1-yl)-1,2-diethyl-1,2-dihydropyrazol-3-one and 2,3-diamino-6-hydroxy-6,7-dihydro-1H,5H-pyrazolo[1,2-a]pyrazol-1-one.

Use will preferably be made of 2,3-diamino-6,7-dihydro-1H,5H-pyrazolo[1,2-a]pyrazol-1-one and/or a corresponding salt.

Heterocyclic bases that will preferably be used are 4,5-diamino-1-(β-hydroxyethyl)pyrazole and/or 2,3-diamino-6,7-dihydro-1H,5H-pyrazolo[1,2-a]pyrazol-1-one and/or 2-[(3-aminopyrazolo[1,5-a]pyridin-2-yl)oxy]ethanol and/or a corresponding salt.

Among these coupling agents that can be used in the invention, mention may be made in particular of meta-phenylenediamines, meta-aminophenols, meta-diphenols, naphthalene-based coupling agents and heterocyclic coupling agents, and also the corresponding addition salts.

Mention may be made, for example, of 1,3-dihydroxybenzene, 1,3-dihydroxy-2-methylbenzene, 4-chloro-1,3-dihydroxybenzene, 2,4-diamino-1-(β-hydroxyethyloxy)benzene, 2-amino-4-(β-hydroxyethylamino)-1-methoxybenzene, 1,3-diaminobenzene, 1,3-bis(2,4-diaminophenoxy)propane, 3-ureidoaniline, 3-ureido-1-dimethylaminobenzene, sesamol, 1-β-hydroxyethylamino-3,4-methylenedioxybenzene, α-naphthol, 2-methyl-1-naphthol, 6-hydroxyindole, 4-hydroxyindole, 4-hydroxy-N-methylindole, 2-amino-3-hydroxypyridine, 6-hydroxybenzomorpholine, 3,5-diamino-2,6-dimethoxypyridine, 1-N-(β-hydroxyethyl)amino-3,4-methylenedioxybenzene, 2,6-bis(β-hydroxyethylamino)toluene, 6-hydroxyindoline, 2,6-dihydroxy-4-methylpyridine, 1-H-3-methylpyrazol-5-one, 1-phenyl-3-methylpyrazol-5-one, 2,6-dimethylpyrazolo[1,5-b]-1,2,4-triazole, 2,6-dimethyl[3,2-c]-1,2,4-triazole and 6-methylpyrazolo[1,5-a]benzimidazole, 2-methyl-5-aminophenol, 5-N-(β-hydroxyethyl)amino-2-methylphenol, 3-aminophenol and 3-amino-2-chloro-6-methylphenol, the corresponding addition salts with an acid and the corresponding mixtures.

In general, the addition salts of oxidation bases and of coupling agents that may be used in the context of the invention are chosen in particular from the addition salts with an acid such as the hydrochlorides, hydrobromides, sulfates, citrates, succinates, tartrates, lactates, tosylates, benzenesulfonates, phosphates and acetates.

When it is (they are) present, the oxidation base(s) each advantageously represent(s) from 0.001% to 10% by weight relative to the total weight of the composition, and preferably from 0.005% to 5% by weight relative to the total weight of the composition and of the ready-to-use composition.

The coupling agent(s), if it is (they are) present, each advantageously represent(s) from 0.001% to 10% by weight relative to the total weight of the composition, and preferably from 0.005% to 5% by weight relative to the total weight of the composition and of the ready-to-use composition.

*b) Direct dyes*

The direct dye(s) b) is (are) generally chosen from synthetic or natural direct dyes, chosen from ionic and nonionic species, preferably anionic, cationic or nonionic species, either as sole dyes or in addition to the oxidation dye(s) a).

Examples of suitable synthetic direct dyes that may be mentioned include azo direct dyes; (poly)methine dyes such as cyanines, hemicyanines and styryls; carbonyl dyes; azine dyes; nitro(hetero)aryl dyes; tri(hetero)arylmethane dyes; porphyrin dyes; phthalocyanine dyes and benzene dyes, alone or in the form of mixtures.

The synthetic direct dye(s) is (are) preferably chosen from neutral, acidic or cationic nitrobenzene direct dyes, neutral, anionic or cationic (poly)azo direct dyes, neutral, anionic or cationic hydrazono direct dyes, neutral, anionic or cationic quinone and in particular anthraquinone direct dyes, azine and (poly)arylmethane direct dyes, (poly)methines such as styryls, porphyrins, phthalocyanines, methine cyanines, and mixtures thereof; more preferentially chosen from (poly)azo, hydrazono, anthraquinone or triarylmethane direct dyes.

Among the cationic synthetic, preferably (poly)azo or hydrazono, direct dyes, mention may in particular be made of Basic Red 51, Basic Yellow 87 and Basic Orange 31.

Among the anionic synthetic, preferably (poly)azo or anthraquinone, direct dyes, mention may in particular be made of Acid Violet 43 and Acid Orange 7.

Among the natural direct dyes that may be used according to the invention, mention may be made of hennotannic acid, gallic acid, juglone, alizarin, purpurin, carminic acid, kermesic acid, laccaic acid, purpurogallin, protocatechaldehyde, indigo, isatin, curcumin, spinulosin, apigenidin and orcein, lawsone, hematein, hematoxylin, brazilin, brazilein, anthocyans, betanin, chlorophyllin, monascus. Extracts or decoctions containing these natural dyes and in particular poultices or extracts based on henna and/or indigo may also be used.

When they are present, the direct dye(s) more particularly represent(s) 0.001% to 25% by weight, preferably 0.005% to 10% by weight, more preferentially from 0.005% to 5% by weight of the total weight of the composition.

The composition according to the invention preferably comprises a cosmetically acceptable medium. For the purposes of the present invention, the term *"cosmetically acceptable medium"* means a medium that is compatible with keratin fibers, and in particular human keratin fibers such as the hair.

The cosmetically acceptable medium of the composition in accordance with the present invention generally comprises water and/or one or more organic, preferably water-soluble, solvents.

Examples of organic solvents that may be mentioned include $C_1$-$C_4$ lower alkanols, such as ethanol and isopropanol; aromatic alcohols such as benzyl alcohol or phenoxyethanol; polyols or polyol ethers such as ethylene glycol monomethyl, monoethyl and monobutyl ethers, propylene glycol or ethers thereof such as propylene glycol monomethyl ether, butylene glycol, pentylene glycol, hexylene glycol, dipropylene glycol, and also diethylene glycol alkyl ethers, for instance diethylene glycol monoethyl ether, monomethyl ether or monobutyl ether, or else glycerol; and also mixtures

thereof.

In one particular embodiment of the invention, the composition comprises one or more organic solvents, preferably chosen from aromatic alcohols, more preferentially chosen from benzyl alcohol and phenoxyethanol, phenylethyl alcohol, better still benzyl alcohol.

The composition is preferably aqueous.

The water content generally ranges from 10% to 99% by weight, preferably from 20% to 98% by weight and better still from 70% to 95% by weight relative to the total weight of the composition.

The organic solvent(s), if it is (they are) present, represent(s) a total content usually ranging from 1% to 40% by weight and preferably from 5% to 30% by weight relative to the weight of the composition.

According to one embodiment, the composition further comprises one or more anionic, cationic, nonionic, amphoteric or zwitterionic surfactants or mixtures thereof. Preferably, the composition comprises one or more nonionic surfactants.

The term *"nonionic surfactant*" means an amphiphilic compound that is not capable of dissociating into ions in aqueous solution.

Several classes of nonionic surfactants may be mentioned:

- alkoxylated and especially ethoxylated derivatives of i) alcohols, ii) alkylphenols, iii) fatty acids, iv) monoalkanolamides, v) sorbitan esters (Spans and Tweens), vi) alkoxylated fatty acid amines and vii) ethylene oxide-propylene oxide copolymers (occasionally referenced as polymeric surfactants);
- polyhydroxylated surfactants such as glycol esters,
- surfactants derived from monosaccharides and polysaccharides,
- glycerol (and polyglycerol) esters,
- glucoside (and polyglucoside) and sucrose esters,
- amine oxide, sulfinyl, sulfoxide and phosphine surfactants.

The alkoxylated surfactants may originate from the products of condensation of hydrophobic compounds such as alcohols, phenols, mercaptans, amines, carboxylic acids or carbonamides with oligoglycol ethers, fatty acid esters of (di)glycerol, of sugars, of hydrogenated sugars such as sorbitol, or alkyl (poly)glucosides.

According to a particular embodiment of the invention, the nonionic surfactant is chosen from alkoxylated and particularly ethoxylated or glycerolated nonionic surfactants, or mixtures thereof.

More particularly, the nonionic surfactant is chosen from:

- oxyalkylenated or glycerolated fatty alcohols;
- oxyalkylenated alkylphenols, the alkyl chain of which is a $C_8$-$C_{18}$ alkyl chain;
- oxyalkylenated or glycerolated fatty amides;
- oxyalkylenated vegetable oils;
- optionally oxyalkylenated $C_6$-$C_{30}$ acid esters of sorbitan;
- optionally oxyalkylenated fatty acid esters of sucrose;
- fatty acid esters of polyethylene glycol;
- ($C_6$-$C_{30}$)alkyl polyglycosides;
- N-(C6-C30 alkyl)glucamine derivatives;
- amine oxides such as ($C_{10}$ - $C_{14}$)alkylamine oxides or N-acylaminopropylmorpholine oxides;
- copolymers of ethylene oxide and propylene oxide;

- mixtures thereof.

More particularly, the mean number of oxyalkylene units is advantageously between 2 and 150 units. Preferably, they are oxyethylene or oxypropylene units or mixtures thereof.

As regards the glycerolated surfactants, they preferably comprise on average 1 to 20 and in particular 1.5 to 5 glycerol groups.

Preferably, the surfactant(s) of use in the invention is (are) nonionic surfactants, preferably chosen from alkyl polyglucosides, more preferentially from $C_8$-$C_{16}$alkyl polyglucosides.

When it is (they are) present, the total content of nonionic surfactant(s) represents from 0.01% to 20% by weight relative to the weight of the composition or compositions containing the nonionic surfactant(s), preferably from 0.1% to 10% by weight relative to the weight of the composition containing it or them.

According to a particular embodiment of the invention, the composition further comprises one or more basifying agents.

These basifying agents are bases that make it possible to increase the pH of the composition(s) in which they are present. The basifying agent is a Brønsted, Lowry or Lewis base. It may be mineral or organic.

Particularly, this alkaline agent is chosen from aqueous ammonia, alkali metal carbonates, alkanolamines, such as monoethanolamine, diethanolamine or triethanolamine, and also derivatives thereof, sodium hydroxide, potassium hydroxide and the compounds of formula (II) below:

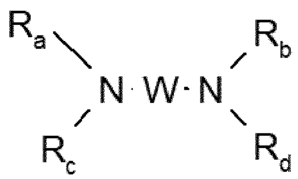

(II)

in which W is a propylene residue optionally substituted with a hydroxyl group or a $C_1$-$C_4$ alkyl radical and $R_a$, $R_b$, $R_c$ and $R_d$, which are identical or different, represent a hydrogen atom or a $C_1$-$C_4$ alkyl or $C_1$-$C_4$ hydroxyalkyl radical.

According to one particular embodiment, the basifying agent(s) is (are) chosen from aqueous ammonia, alkanolamines and amino acids.

According to one particular embodiment, the basifying agent(s) is (are) chosen from organic alkaline agents, preferably from alkanolamines and amino acids, preferably from alkanolamines, preferably monoethanolamine.

When it is (they are) present, the basifying agent(s) as defined previously represent(s) from 0.001% to 10% by weight of the weight of the composition(s) containing them, more particularly from 0.005% to 8% by weight of the composition.

According to a particular embodiment of the invention, the composition further comprises one or more chemical oxidizing agents.

The term *"chemical oxidizing agent"* means an oxidizing agent other than atmospheric oxygen.

More particularly, the oxidizing agent(s) is (are) chosen from hydrogen peroxide, urea peroxide, alkali metal bromates, peroxygenated salts, for instance persulfates or perborates, peracids and precursors thereof and alkali metal or alkaline-earth metal percarbonates.

The oxidizing agent is advantageously hydrogen peroxide.

The concentration of oxidizing agents may range more particularly from 0.1% to 20% by weight, even more preferentially from 0.5% to 15% by weight and better still from 1% to 10% by weight relative to the weight of the composition.

When the composition of the invention contains one or more dye precursors, the composition preferentially contains one or more alkaline agents.

The composition of the invention may be mixed at the time of use with an oxidizing agent. This embodiment is particularly preferred when the composition of the invention comprises a dye precursor such as an oxidation base and/or a coupler.

***The adjuvants:***

The composition according to the invention may also contain various ingredients conventionally used in hair dye compositions, such as polymers other than the polysaccharide polymers of use in the present invention and which may be anionic, cationic, nonionic, amphoteric or zwitterionic polymers or mixtures thereof; inorganic thickeners, and in particular fillers such as clays or talc; organic thickeners other than polysaccharides, in particular anionic, cationic, nonionic and amphoteric polymeric associative thickeners; antioxidants; penetrants; sequestrants; fragrances; dispersants; film-forming agents; ceramides; preserving agents; opacifiers; anionic, cationic, amphoteric and nonionic surfactants.

The above adjuvants are generally present in an amount of for each of them between 0.01% and 40% by weight relative to the weight of the composition or compositions comprising the ingredients as defined above, preferably between 0.1% and 20% by weight relative to the weight of the composition.

Needless to say, those skilled in the art will take care to select this or these optional adjuvants such that the advantageous properties intrinsically associated with the composition in accordance with the invention are not, or are not substantially, adversely affected by the envisioned addition(s).

The composition according to the invention may be in various forms, such as in the form of a cream, a gel, a mousse or a milk or in any other form that is suitable for dyeing human keratin fibers such as the hair.

According to a particular embodiment of the invention, the pH of the composition ranges from 2 to 12, preferably from 2.5 to 10.5.

The pH of these compositions may be adjusted to the desired value by means of acidifying or basifying agents such as those described above usually used in the dyeing of keratin fibers, or alternatively using standard buffer systems.

Mention may be made, among the acidifying agents for the compositions used in the invention, by way of example, of mineral or organic acids, such as hydrochloric acid, orthophosphoric acid, sulfuric acid, carboxylic acids, such as acetic acid, tartaric acid, citric acid or lactic acid, or sulfonic acids. As alkaline agents, use may be made of the alkaline agents described previously.

***Process of the invention***

Another subject of the present invention is a process for dyeing keratin fibers, and in particular human keratin fibers such as the hair, using the composition of the invention as defined above.

According to a particular embodiment of the invention, the dyeing process is performed by applying to the keratin fibers at least one composition comprising:

- one or more polysaccharide-type polymers,

- one or more fatty ethers that are solid at room temperature and at atmospheric pressure, as described previously,

- one or more direct dyes and/or dye precursors.

The keratin fibers may or may not be moistened prior to the application.

According to one particular embodiment of the invention, the process for dyeing keratin fibers is performed in a single step by applying to the keratin fibers the dye composition comprising i), ii) and iii) as defined previously.

The leave-on time after application is generally set at between 3 and 120 minutes, preferentially between 10 and 60 minutes and more preferentially between 15 and 45 minutes.

The application temperature is generally between room temperature (15 to 30°C) and 80°C and more particularly between 15 and 45°C.

A particular embodiment of the invention relates to a dyeing process which is performed at room temperature (25°C).

In all the particular embodiments and variants of the processes described previously, the compositions mentioned are ready-to-use compositions which can result from the extemporaneous mixing of two or more compositions and in particular of compositions present in dyeing kits.

The examples that follow serve to illustrate the invention without, however, being limiting in nature.

**Example**

Example 1

The following compositions are prepared (amounts in g% of active material):

[Table 1]

| | **A1 (invention)** | **A2 (comparative)** |
|---|---|---|
| **Basic Orange 31** | 0.5 | 0.5 |
| **Benzyl alcohol** | 2 | 2 |

(continued)

| | A1 (invention) | A2 (comparative) |
|---|---|---|
| **Ethyl alcohol as a 95% aqueous solution** | 10 | 10 |
| **Distearyl ether** | 10 | 10 |
| **Sclerotium gum (INCI name)** | 1 | - |
| **Ammonium acryloyldimethyltaurate/VP copolymer (INCI Name)** | - | 1 |
| **Coco-glucoside ((C8-C16)alkyl polyglucoside (1,4) as a 53% aqueous solution)** | 1 | 1 |
| **Water** | Q.s. for 100 | Q.s. for 100 |
| **pH** | 6 ± 0.2 | 6 ± 0.2 |

Compositions A1 and A2 are applied to locks of natural dry hair containing 90% white hair and to locks of permanent-waved dry hair containing 90% white hair in an amount of 5 g of composition per 1 g of hair.

After a leave-on time of 30 minutes on a hot plate at 33°C under plastic wrap, the locks are rinsed with water then shampooed and dried.

*Colorimetric results:*

The coloration of the hair is evaluated visually and read on a Minolta spectrocolorimeter (CM3600d, illuminant D65, angle 10°, SCI values) for the L*, a*, b* colorimetric measurements.

In this L*a*b* system, L* represents the intensity of the color, a* indicates the green/red color axis and b* indicates the blue/yellow color axis. The lower the value of L*, the darker or more intense the color. The higher the value of a*, the redder the shade, and the higher the value of b*, the yellower the shade.

*Selectivity*

The variation in coloration between the dyed locks of natural/permanent-waved hair is defined by (ΔE*) according to the following equation:

$$\Delta E^* = \sqrt{(L^* - L_o^*)^2 + (a^* - a_o^*)^2 + (b^* - b_o^*)^2}$$

In this equation, L*, a* and b* represent the values measured after dyeing locks of natural hair, and L0*, a0* and b0* represent the values measured on locks of permanent-waved hair.

The higher the value of ΔE, the greater the difference in color between the natural and permanent-waved locks. The locks of permanent-waved hair are representative of the tips of hair whereas the locks of natural hair are representative of the hair at the root. A low ΔE difference is representative of a more uniform coloring between the roots and the tips of the hair.

The results are given in the table below:

[Table 2]

| | Color | ΔE* |
|---|---|---|
| **composition A1 (invention)** | Highly chromatic orange | 1.7 |
| **composition A2 (comparative)** | Chromatic orange | 5.9 |

Locks dyed a highly chromatic orange and with a lower selectivity were obtained in the case of the composition A1 as confirmed by the colorimetric measurements of ΔE.

The composition according to the invention (A1) results in a more homogeneous coloring (markedly lower selectivity), it also makes it possible to color the hair more chromatically than the comparative composition (A2).

Example 2

The following compositions are prepared (amounts in g% of active material):

[Table 3]

| | B1 (invention) | B2 (comparative) | C1 (invention) | C2 (comparative) |
|---|---|---|---|---|
| **Acid Blue 25** | 0.5 | 0.5 | - | - |
| **HC Blue 15** | - | - | 0.5 | 0.5 |
| **Benzyl alcohol** | 2 | 2 | 2 | 2 |
| **Ethyl alcohol as a 95% aqueous solution** | 10 | 10 | 10 | 10 |
| **Distearyl ether** | 10 | 10 | 10 | 10 |
| **Sclerotium gum (INCI name)** | 1 | - | 1 | - |
| **Ammonium acryloyldimethyltaurate/VP copolymer (INCI Name)** | - | 1 | - | 1 |
| **Coco-glucoside ((C8-C16)alkyl polyglucoside (1,4) as a 53% aqueous solution)** | 1 | 1 | 1 | 1 |
| **Citric acid** | 2 | 2 | - | - |
| **Water** | Q.s. for 100 | Q.s. for 100 | Q.s. for 100 | Q.s. for 100 |
| **pH** | 2.6 ± 0.2 | 2.6 ± 0.2 | 6 ± 0.2 | 6 ± 0.2 |
| **Appearance** | homogeneous | **aggregates** | homogeneous | homogeneous |

Homogeneous smooth and shiny cream gels that are very easy to apply and to rinse are obtained in the case of the compositions according to the invention (B1, C1) whereas in the case of the comparative composition B2 the presence of aggregates (inhomogeneous formula) is noted.
Compositions B1, B2, C1 and C2 are applied to locks of permanent-waved dry hair containing 90% white hair, with a bath ratio of 5 g of composition per 1 g of hair.
After a leave-on time of 30 minutes on a hot plate at 33°C under plastic wrap, the locks are rinsed with water then shampooed and dried.

*Colorimetric results:*

The coloration is evaluated as described in example 1.
The increase in coloration is represented by ΔE* in the table below which corresponds to the difference in coloration between the untreated locks and the dyed locks.
The variation in coloration between the dyed locks of permanent-waved white hair and the locks before treatment are defined by (ΔE*) according to the following equation:

$$\Delta E^* = \sqrt{(L^* - L_o^*)^2 + (a^* - a_o^*)^2 + (b^* - b_o^*)^2}$$

In this equation, L*, a* and b* represent the values measured after dyeing permanent-waved hair, and L0*, a0* and b0* represent the values measured on untreated permanent-waved hair.
The higher the value of ΔE, the greater the difference in color between the control locks and the dyed locks.
L* represents the lightness; the lower the value of L*, the more intense the coloring obtained.
The results are given in the table below:

[Table 4]

| | L* (D65) | a* (D65) | b* (D65) | ΔE* (increase in coloration) |
|---|---|---|---|---|
| **Permanent-waved hair before treatment** | 69.26 | 0.69 | 17.7 | - |
| **Permanent-waved hair after treatment with B1 (invention)** | 30.65 | -2.47 | -26.58 | **58.8** |
| **Permanent-waved hair after treatment with B2 (comparative)** | 34.08 | -6.37 | -25.27 | 55.9 |
| **Permanent-waved hair after treatment with C1 (invention)** | 27.53 | 3.99 | -31.3 | **64.5** |
| **Permanent-waved hair after treatment with C2 (comparative)** | 34.41 | -6.9 | -30.81 | 60.2 |

More intense blue locks were obtained in the case of the compositions of the invention B1 and C1 in comparison with the comparative compositions respectively B2 and C2.

The compositions according to the invention (B1, C1) result in significantly higher values of ΔE, therefore in a better increase in the color than the comparative compositions B2 and C2 respectively.

Furthermore, the compositions according to the invention (B1, C1) result in significantly lower values of L*, therefore in a more intense coloration, compared to the comparative compositions B2 and C2 respectively.

Example 3

The following compositions are prepared (amounts in g% of active material):

[Table 5]

| | A1 (invention) | A2 (comparative) |
|---|---|---|
| **Basic Red 51** | 0.5 | 0.5 |
| **Benzyl alcohol** | 2 | 2 |
| **Ethyl alcohol as a 95% aqueous solution** | 10 | 10 |
| **Distearyl ether/Dicetyl ether (1 :1)** | **15** | **-** |
| **Xanthane gum** | **1** | **1** |
| **Coco-glucoside ((C8-C16)alkyl polyglucoside (1,4) as a 53% aqueous solution)** | 1.5 | 1.5 |
| **Water** | Q.s. for 100 | Q.s. for 100 |
| **pH** | 8.7 ± 0.2 | 8.6 ± 0.2 |

Compositions A1 and A2 are applied to permanent-waved dry hair containing 90% white hair in an amount of 5 g of composition per 1 g of hair.

After a leave-on time of 30 minutes on a hot plate at 33°C under plastic wrap, the locks are rinsed with water then shampooed and dried.

*Colorimetric results:*

The coloration is evaluated as described in example 1.

The chromaticity is calculated according to the following formula: The higher the value of the chromaticity C *, the more the color of the fibers is chromatic.

$$C* = \sqrt{(a*)^2 + (b*)^2}$$

The results are given in the table below:

[Table 6]

| | a* (D65) | b* (D65) | C* (D65) |
|---|---|---|---|
| **Permanent-waved hair before treatment** | 0.74 | 14.66 | - |
| **treatment with A1 (invention)** | 35.58 | 13.88 | **38.2** |
| **treatment with A2 (comparative)** | 30.05 | 10.08 | 31.7 |

The fibers treated with composition A according to the invention found to have a more chromatic color (higher C * value) compared to those treated with composition B according to the prior art, while ending up with good intensity color.

**Claims**

**1.** A dye composition comprising:

- at least one polysaccharide-type polymer,
- at least one fatty ether that is solid at room temperature and atmospheric pressure, of formula R-O-R' with R and R', which are identical or different, denoting a linear or branched alkyl or alkenyl radical, R and R' comprising at least 12 carbon atoms, and
- at least one direct dye and/or at least one dye precursor.

**2.** The composition as claimed in any one of the preceding claims, wherein the polysaccharide-type polymer(s) is (are) nonionic.

**3.** The composition as claimed in any one of the preceding claims, wherein the polysaccharide-type polymer(s) are chosen from microbial gums, preferably from scleroglucan gums.

**4.** The composition as claimed in any one of the preceding claims, wherein the polymer(s) is (are) present in a total amount ranging from 0.001% to 10% by weight, preferably from 0.05% to 5% by weight, preferentially from 0.1% to 3% by weight and better still from 0.5% to 2.5% by weight relative to the total weight of the composition.

**5.** The composition as claimed in any one of the preceding claims, wherein the R and R' radicals, which are identical or different, denote a $C_{12}$-$C_{40}$, preferably $C_{12}$-$C_{30}$, more preferentially $C_{14}$-$C_{24}$ linear or branched alkyl or alkenyl radical.

**6.** The composition as claimed in any one of the preceding claims, wherein R and R', which are identical or different, represent a $C_{14}$-$C_{24}$ linear alkyl radical, preferably R and R' are identical and represent a $C_{14}$-$C_{24}$, better still $C_{16}$-$C_{22}$, linear alkyl radical.

**7.** The composition as claimed in any one of the preceding claims, wherein the solid fatty ether is distearyl ether.

**8.** The composition as claimed in any one of the preceding claims, wherein the solid fatty ether(s) is (are) present in a total amount ranging from 0.1% to 40% by weight, preferably ranging from 1% to 25% by weight, preferentially ranging from 5% to 15% by weight relative to the total weight of the composition.

**9.** The composition as claimed in any one of the preceding claims, wherein the dye precursor(s) is (are) chosen from oxidation bases and/or couplers.

**10.** The composition as claimed in any one of the preceding claims, wherein the direct dye(s) is (are) chosen from a (some) anionic, cationic or neutral synthetic direct dye(s), preferably chosen from neutral, acidic or cationic nitrobenzene direct dyes, neutral, anionic or cationic (poly)azo direct dyes, neutral, anionic or cationic hydrazono direct dyes, neutral, anionic or cationic quinone and in particular anthraquinone direct dyes, azine and (poly)arylmethane direct dyes, (poly)methines such as styryls, porphyrins, phthalocyanines, methine cyanines, and mixtures thereof; more preferentially chosen from (poly)azo, hydrazono, anthraquinone or triarylmethane direct dyes.

**11.** The composition as claimed in any one of the preceding claims, wherein the direct dye(s) is (are) chosen from natural dye(s).

**12.** The composition as claimed in any one of the preceding claims, wherein the direct dye(s) is (are) chosen from anionic, nonionic or cationic direct dyes.

**13.** The composition as claimed in any one of the preceding claims, wherein the direct dye(s) is (are) present in a total amount of between 0.001% and 25% by weight, preferably 0.005% to 10% by weight, preferentially from 0.005% to 5% by weight of the total weight of the composition.

**14.** The composition as claimed in any one of the preceding claims which comprises one or more surfactants chosen from anionic, cationic, nonionic, amphoteric and zwitterionic surfactants or mixtures thereof, particularly chosen from nonionic surfactants, preferably chosen from alkyl polyglucosides.

**15.** The composition as claimed in any one of the preceding claims which comprises one or more organic solvents.

**16.** A process for dyeing keratin fibers which comprises at least the step of applying the composition as claimed in any one of the preceding claims on the keratin fibers.

**17.** The use of a composition as claimed in one of claims 1 to 15 for dyeing keratin fibers. ]

## Patentansprüche

**1.** Färbezusammensetzung, umfassend:

- mindestens ein Polymer vom Polysaccharid-Typ,
- mindestens einen bei Raumtemperatur und Normaldruck festen Fettether der Formel R-O-R', wobei R und R', die gleich oder verschieden sind, für einen linearen oder verzweigten Alkyl- oder Alkenylrest stehen, wobei R und R' mindestens 12 Kohlenstoffatome umfassen, und
- mindestens einen Direktfarbstoff und/oder mindestens eine Farbstoffvorstufe.

**2.** Zusammensetzung nach einem der vorhergehenden Ansprüche, wobei das Polymer bzw. die Polymere vom Polysaccharid-Typ nichtionisch ist bzw. sind.

**3.** Zusammensetzung nach einem der vorhergehenden Ansprüche, wobei das Polymer bzw. die Polymere vom Polysaccharid-Typ aus mikrobiellen Gummen, vorzugsweise aus Skleroglucan-Gummen, ausgewählt ist bzw. sind.

**4.** Zusammensetzung nach einem der vorhergehenden Ansprüche, wobei das Polymer bzw. die Polymere in einer Gesamtmenge im Bereich von 0,001 bis 10 Gew.%, vorzugsweise von 0,05 bis 5 Gew.-%, bevorzugt von 0,1 bis 3 Gew.-% noch besser von 0,5 bis 2,5 Gew.%, bezogen auf das Gesamtgewicht der Zusammensetzung, vorliegt bzw. vorliegen.

**5.** Zusammensetzung nach einem der vorhergehenden Ansprüche, wobei die Reste R und R', die gleich oder verschieden sind, für einen linearen oder verzweigten $C_{12}$-$C_{40}$-, vorzugsweise $C_{12}$-$C_{30}$- und weiter bevorzugt $C_{14}$-$C_{24}$-Alkyl- oder -Alkenylrest stehen.

**6.** Zusammensetzung nach einem der vorhergehenden Ansprüche, wobei die Reste R und R’, die gleich oder verschieden sind, für einen linearen $C_{14}$-$C_{24}$-Alkylrest stehen, vorzugsweise R und R’ identisch sind und für einen linearen $C_{14}$-$C_{24}$- und noch besser $C_{16}$-$C_{22}$-Alkylrest stehen.

**7.** Zusammensetzung nach einem der vorhergehenden Ansprüche, wobei es sich bei dem festen Fettether um Distearylether handelt.

**8.** Zusammensetzung nach einem der vorhergehenden Ansprüche, wobei der feste Fettether bzw. die festen Fettether in einer Gesamtmenge im Bereich von 0,1 bis 40 Gew.-%, vorzugsweise im Bereich von 1 bis 25 Gew.-%, bevorzugt im Bereich von 5 bis 15 Gew.-%, bezogen auf das Gesamtgewicht der Zusammensetzung, vorliegt bzw. vorliegen.

**9.** Zusammensetzung nach einem der vorhergehenden Ansprüche, wobei die Farbstoffvorstufe bzw. die Farbstoffvorstufen aus Oxidationsbasen und/oder Kupplern ausgewählt ist bzw. sind.

**10.** Zusammensetzung nach einem der vorhergehenden Ansprüche, wobei der Direktfarbstoff bzw. die Direktfarbstoffe aus einem oder mehreren anionischen, kationischen oder neutralen synthetischen Direktfarbstoffen ausgewählt ist bzw. sind, der bzw. die vorzugsweise aus neutralen, sauren oder kationischen Nitrobenzol-Direktfarbstoffen, neutralen, anionischen oder kationischen (Poly)azo-Direktfarbstoffen, neutralen, anionischen oder kationischen Hydrazono-Direktfarbstoffen, neutralen, anionischen oder kationischen Chinon- und insbesondere Anthrachinon-Direktfarbstoffen, Azin- und (Poly)arylmethan-Direktfarbstoffen, (Poly)-methinen wie Styrylen, Porphyrinen, Phthalocyanin, Methincyaninen und Mischungen davon ausgewählt ist bzw. sind und weiter bevorzugt aus (Poly)azo-, Hydrazono-, Anthrachinon- oder Triarylmethan-Direktfarbstoffen ausgewählt ist bzw. sind.

**11.** Zusammensetzung nach einem der vorhergehenden Ansprüche, wobei der Direktfarbstoff bzw. die Direktfarbstoffe aus einem natürlichen Farbstoff bzw. natürlichen Farbstoffen ausgewählt ist bzw. sind.

**12.** Zusammensetzung nach einem der vorhergehenden Ansprüche, wobei der Direktfarbstoff bzw. die Direktfarbstoffe aus anionischen, nichtionischen oder kationischen Direktfarbstoffen ausgewählt ist bzw. sind.

**13.** Zusammensetzung nach einem der vorhergehenden Ansprüche, wobei der Direktfarbstoff bzw. die Direktfarbstoffe in einer Gesamtmenge zwischen 0,001 und 25 Gew.-%, vorzugsweise 0,005 bis 10 Gew.%, bevorzugt 0,005 bis 5 Gew.-%, der Gesamtmenge der Zusammensetzung vorliegt bzw. vorliegen.

**14.** Zusammensetzung nach einem der vorhergehenden Ansprüche, die ein oder mehrere Tenside umfasst, die aus anionischen, kationischen, nichtionischen, amphoteren und zwitterionischen Tensiden oder Mischungen davon und insbesondere aus nichtionischen Tensiden, vorzugsweise aus Alkylpolyglucosiden, ausgewählt ist.

**15.** Zusammensetzung nach einem der vorhergehenden Ansprüche, die ein oder mehrere organische Lösungsmittel umfasst.

**16.** Verfahren zum Färben von Keratinfasern, das mindestens den Schritt des Aufbringens der Zusammensetzung nach einem der vorhergehenden Ansprüche auf die Keratinfasern umfasst.

**17.** Verwendung einer Zusammensetzung nach einem der Ansprüche 1 bis 15 zum Färben von Keratinfasern.

## Revendications

**1.** Composition colorante comprenant :

- au moins un polymère de type polysaccharide,
- au moins un éther gras qui est solide à température ambiante et pression atmosphérique, de formule R-O-R’ avec R et R’, qui sont identiques ou différents, désignant un radical alkyle ou alcényle, linéaire ou ramifié, R et R’ comprenant au moins 12 atomes de carbone, et
- au moins un colorant direct et/ou au moins un précurseur de colorant.

**2.** Composition selon l'une quelconque des revendications précédentes, dans laquelle le ou les polymère(s) de type polysaccharide est (sont) non ionique(s).

**3.** Composition selon l'une quelconque des revendications précédentes dans laquelle le ou les polymère(s) de type polysaccharide sont choisis parmi des gommes microbiennes, de préférence parmi des gommes de scléroglucane.

**4.** Composition selon l'une quelconque des revendications précédentes dans laquelle le ou les polymère(s) est(sont) présent(s) en une quantité totale allant de 0,001 % à 10 % en poids, de préférence de 0,05 % à 5 % en poids, préférentiellement de 0,1 % à 3 % en poids et mieux encore de 0,5 % à 2,5 % en poids par rapport au poids total de la composition.

**5.** Composition selon l'une quelconque des revendications précédentes dans laquelle les radicaux R et R', qui sont identiques ou différents, désignent un radical alkyle ou alcényle, linéaire ou ramifié, en $C_{12}$-$C_{40}$, de préférence en $C_{12}$-$C_{30}$, plus préférentiellement en $C_{14}$-$C_{24}$.

**6.** Composition selon l'une quelconque des revendications précédentes dans laquelle R et R', qui sont identiques ou différents, représentent un radical alkyle linéaire en $C_{14}$-$C_{24}$, de préférence R et R' sont identiques et représentent un radical alkyle linéaire en $C_{14}$-$C_{24}$, mieux encore en $C_{16}$-$C_{22}$.

**7.** Composition selon l'une quelconque des revendications précédentes dans laquelle l'éther gras solide est le distéaryléther.

**8.** Composition selon l'une quelconque des revendications précédentes, dans laquelle le ou les éther(s) gras solide(s) est (sont) présent(s) en une quantité totale allant de 0,1 % à 40 % en poids, de préférence allant de 1 % à 25 % en poids, préférentiellement allant de 5 % à 15 % en poids par rapport au poids total de la composition.

**9.** Composition selon dans l'une quelconque des revendications précédentes dans laquelle le ou les précurseur(s) de colorant est (sont) choisi(s) parmi les bases d'oxydation et/ou les coupleurs.

**10.** Composition selon l'une quelconque des revendications précédentes dans laquelle le ou les colorant(s) direct(s) est (sont) choisi(s) parmi un (des) colorant(s) direct(s) synthétique(s) anionique(s), cationique(s) ou neutre(s), de préférence choisi(s) parmi des colorants directs de nitrobenzène neutres, acides ou cationiques, des colorants directs (poly)azoïques neutres, anioniques ou cationiques, des colorants directs hydrazono neutres, anioniques ou cationiques, des colorants directs de type quinone et en particulier anthraquinonique neutres, anioniques ou cationiques, des colorants directs de type azine et (poly)arylméthane, des (poly)méthines tels que les styryles, les porphyrines, les phtalocyanines, les cyanines de méthine, et des mélanges correspondants ; plus préférentiellement choisis parmi des colorants directs (poly)azoïques, hydrazono, anthraquinone ou triarylméthane.

**11.** Composition selon l'une quelconque des revendications précédentes dans laquelle le ou les colorant(s) direct(s) est (sont) choisi(s) parmi un ou des colorant(s) naturel(s).

**12.** Composition selon l'une quelconque des revendications dans laquelle le ou les colorant(s) direct(s) est (sont) choisi(s) parmi les colorants directs anioniques, non ioniques ou cationiques.

**13.** Composition selon l'une quelconque des revendications précédentes dans laquelle le ou les colorant(s) direct(s) est (sont) présents en une quantité totale comprise entre 0,001 % et 25 % en poids, de préférence de 0,005 % à 10 % en poids, préférentiellement de 0,005 % à 5 % en poids du poids total de la composition.

**14.** Composition selon l'une quelconque des revendications précédentes qui comprend un ou plusieurs tensioactifs choisis parmi des tensioactifs anioniques, cationiques, non ioniques, amphotères et zwitterioniques ou des mélanges correspondants, particulièrement choisis parmi des tensioactifs non ioniques, de préférence choisis parmi des alkylpolyglucosides.

**15.** Composition selon l'une quelconque des revendications précédentes qui comprend un ou plusieurs solvant(s) organique(s).

**16.** Procédé pour la coloration de fibres kératiniques qui comprend au moins l'étape d'application de la composition selon l'une quelconque des revendications précédentes sur les fibres kératiniques.

**17.** Utilisation d’une composition selon l’une des revendications 1 à 15 pour la coloration de fibres kératiniques.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4131576 A
- FR 2633940
- DE 4127230
- GB 1026978 A
- GB 1153196 A
- FR 2801308
- DE 2359399
- JP 63169571 A
- JP 5063124 A
- EP 0770375 A
- WO 9615765 A
- DE 3843892
- DE 4133957
- WO 9408969 A
- WO 9408970 A
- FR 2733749 A
- DE 19543988
- FR 2886136 A